# EUROPÄISCHE PATENTANMELDUNG

(11) **EP 0 958 811 A1**
(43) Veröffentlichungstag der Anmeldung: **24.11.1999**
(21) Anmeldenummer: 99109059.8
(22) Anmeldetag: 07.05.1999
(51) Int. Cl.: A61K 7/48, C08L 77/10, C11D 3/37

(54) **Hydrophob modifizierte Polyasparaginsäurederivate in O/W-Emulsionen**

(30) Priorität: 20.05.1998 DE 19822601
(71) Anmelder: Th. Goldschmidt AG, 45127 Essen (DE)
(72) Erfinder: Grüning, Burghard Dr., 45134 Essen (DE); Simpelkamp, Jörg Dr., 45130 Essen (DE); Weitemeyer, Christian Dr., 45134 Essen (DE)

(57) **Zusammenfassung**

Diese Erfindung beschreibt den Einsatz von Polyasparaginsäurederivaten, weiche mit Alkylresten mit 6 bis 30 C-Atomen hydrophob modifiziert sind, in kosmetischen O/W-Emulsionen, welche hydratgelbildende Konsistenzgeber enthalten.

Die kosmetischen O/W-Emulsionen enthalten ein oder mehrere hydrophob modifizierte Polyasparaginsäurederivate, einen oder mehrere Konsistenzgeber, sowie gegebenenfalls weitere Coemulgatoren und übliche Hilfs- und Zusatzstoffe.

## Beschreibung

Diese Erfindung beschreibt den Einsatz von Polyasparaginsäurederivaten, weiche mit Alkyl- oder Alkenylresten mit 6 bis 30 C-Atomen hydrophob modifiziert sind, in kosmetischen O/W-Emulsionan, weiche hydratgelbildende Konsistenzgeber enthalten.

Polyaminosäurederivate, insbesondere Polyasparaginsäure, haben in jüngster Zeit aufgrund ihrer Eigenschaften besondere Aufmerksamkeit gefunden. Es werden u. a. Anwendungen als biologisch abbaubare Komplexierungsmittel, Enthärter und Waschmittel-Builder vorgeschlagen. Polyasparaginsäure wird i. A. durch alkalische Hydrolyse der unmittelbaren Synthesevorstufe Polysuccinimid (PSI, Anhydropolyasparaginsäure), dam cyclischen Imid der Polyasparaginsäure gewonnen. PSI kann beispielsweise nach EP 0 578 449 A, WO 92/14753, EP 0 659 875 A oder DE 44 20 642 A aus Asparaginsäure hergestellt werden oder ist beispielsweise nach DE 36 26 672 A, EP 0 612 784 A, DE 43 00 020 A oder US 5 219 952 A aus Maleinsäurederivaten und Ammoniak zugänglich. Für diese üblichen Polyasparaginsäuren werden u. a. Anwendungen als Inkrustationsinbibitor, Builder in Waschmitteln, Düngemitteladditiv und Hilfsstoff in der Gerberei vorgeschlagen.

Die von verschiedenen Arbeitsgruppen beschriebene Umsatzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Neri et al. führen die Ringöffnung von PSI mit Ethanolamin durch und erhalten Hydroxyethylaspartamide (J. Med. Chem. 1973, 16, 893-897, Macromol. Synth. 1982, 3, 25-29). DE 37 00 128 A und ER 0 458 079 A beschreiben die nachfolgende Veresterung derartiger Hydroxyethylderivate mit Carbonsäurederivaten und die Verwendung der Produkte in Ultraschallkontrastmitteln und in Wirkstoffdepotzubereitungen.

In FR 24 24 292 A wird die Herstellung von PSI aus Asparaginsäure, die nachfolgende Modifizierung mit substituierten Aminen zu oberflächenaktiven Polyasparaginsäurederivaten und der Einsatz in der Haarkosmetik zur Verbesserung der Kämmbarkeit und des Sitzes beschrieben sowie der Einsatz in Cremes und Lotionen. Auch die nach DE 23 39 369 A zugänglichen Polyasparaginsäurederivate mit sulfonatsubstituierten N-Alkylaspartamideinheiten werden in der Haarkosmetik zur Verbesserung der Kämmbarkeit und des Sitzes eingesetzt.

DE 22 53 190 A beschreibt die Herstellung von oberflächenaktiven Polyaspartamiden durch Öffnung von PSI mit primären und sekundären Aminen.

EP 0 685 504 A beschreibt die Herstellung von nicht hautreizenden Polymeren für kosmetische Anwendungen durch Ringöffnung von PSI mit Aminosäureestern. Der Einsatz von komplexen Grundstoffen wie Aminosäureestern bringt allerdings Nachteile bezüglich des Syntheseaufwandes und der Wirtschaftlichkeit mit sich.

DE 195 24 097 A1 beschreibt Tensidzubereitungen aus Alkyl- oder Alkenylpolyglucosiden und/oder Fettsäure-N-alkylpolyhydroxyalkylamiden im Gemisch mit hydrophobierten Oligopeptiden, beispielsweise Fettsäure-Eiweiß-Kondensationsprodukten. In der gleichen Schrift wird die Herstellung von Veresterungsprodukten der Polyasparaginsäure mit Fettalkoholen in bekannter Weise erwähnt. Während homogene Umsetzungsprodukte, hergestellt unter den üblichen Veresterungsbedingungen, nur schwierig hergestellt werden können, sind copolymere hydrophob modifizierte Polyasparaginsäureester auf Basis von Maleinsäuremonoestern und Ammoniak oder auf Basis von Polysuccinimid und Alkoholen leicht zugänglich, wie aus DE 195 45 678 beziehungsweise der EP 96 118 806.7 hervorgeht.

Fettsäure-Eiweiß-Kondensationsprodukte sind dem Fachmann aus zahlreichen Anwendungen als Tenside wohlbekannt. Aufgrund ihrer naturnahen Struktur auf Polyaminosäurebasis weisen diese ein ausgesprochen mildes und hautfreundliches Verhalten auf. Gerade unter dem Aspekt des zunehmenden Umweltbewußtseins sind derartige Systeme mit naturnaher Grundstruktur interessant speziell für leave-on Kosmetikprodukte. Allerdings besitzen sie nur mäßige Emulgiereigenschaften. Polymere Emulgatoren, wie beispielsweise die in DE 34 36 177 A, EP 0 459 705 A oder US 5 449 510 A beschriebenen Siliconpolyether, liefern stabile Emulsionen, basieren allerdings nicht auf den gewünschten naturnahen Grundstrukturen und sind schlecht oder nicht biologisch abbaubar. Zudem ist dem Fachmann bekannt, daß derartige polymere Emulgatoren nur wenig in der Lage sind, die viskositätserhöhenden und stabilitätsfördernden Wechselwirkungen mit niedermolekularen Konsistenzgebern, wie sie in O/W-Emulsionen verbreitet eingesetzt werden, einzugehen.

Es ist daher die Aufgabe der Erfindung, effektive polymere O/W-Emulgatoren mit naturnahem Grundgerüst zur Verfügung zu stellen.

Die vorgenannte Aufgabe wird gelöst durch kosmetische O/W-Emulsionen, enthaltend ein oder mehrere hydrophob modifizierte Polyasparaginsäurederivate, einen oder mehrere Konsistenzgeber, sowie gegebenenfalls weitere Coemulgatoren und übliche Hilfs- und Zusatzstoffe, insbesondere durch den Einsatz von Polyasparaginsäurederivaten, welche mit Alkyl- oder Alkenylresten mit 6 bis 30 C-Atomen hydrophob modifiziert sind.

Überraschenderweise zeigte sich, daß die erfindungsgemäß eingesetzten Polyasparaginsäurederivate im Gegensatz zu üblichen polymeren Emulgatoren besonders effektiv in Kombination mit konsistenzgebenden hydrophilen Wachsen eingesetzt werden können.

Erfindungsgemäß eingesetzte Polyasparaginsäurederivate sind beispielsweise die durch Umsetzung von Maleinsäuremonoestern mit Ammoniak zugänglichen copolymeren Polyasparaginsäureester, weiche durch eine Polyasparaginsäurestruktur gekennzeichnet sind, bei der die Seitenketten partiell als freie Carbonsäure- bzw. Carboxylatgruppen vorliegen und partiell mit einem oder mehreren Alkoholen mit 1 bis 30 C-Atomen, darunter mindestens ein langkettiger Fettalkohol mit 6 bis 30 C-Atomen, vorzugsweise mit 10 bis 22 C-Atomen, oder dessen Derivaten verestert sind. Diese Copolymeren bestehen zu wenigstens 75 Mol-% der vorhandenen Einheiten aus Struktureinheiten der allgemeinen Formeln (I) und (II), wobei A ein trifunktionelles Kohlenwasserstoffradikal mit 2 C-Atomen der Struktur (A1) oder (A2) ist, 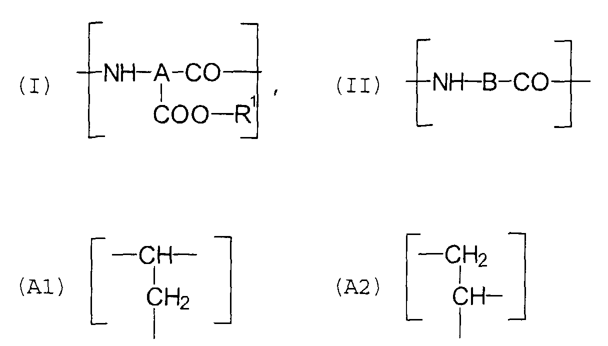
worin R¹ die Bedeutung von R², R³ und R⁴ hat, wobei
R² für ein oder mehrere Reste aus der Gruppe der Alkali-, Erdalkalimetalle, Wasserstoff oder Ammonium, [NR⁵R⁶R⁷R⁸]⁺, worin R⁵ bis R⁸ unabhängig voneinander Wasserstoff, Alkyl oder Alkenyl mit 1 bis 22 C-Atomen oder Hydroxyalkyl mit 1 bis 22 C-Atomen und 1 bis 6 Hydroxygruppen ist,
R³ für gleiche oder verschiedene, geradkettige oder verzweigte, gesättigte oder ungesättigte Alkyl- oder Alkenylreste R⁹ mit 6 bis 30 C-Atomen oder Radikale der Struktur -X-R⁹, wobei X eine Oligo- oder Polyoxyalkylenkette mit 1 bis 100 Oxyalkyleneinheiten ist, und
R⁴ für gleiche oder verschiedene, geradkettige oder verzweigte, gesättigte oder ungesättigte Alkyl- oder Alkenylreste mit 1 bis 5 C-Atomen steht,
und wenigstens jeweils ein Rest R¹ die Bedeutung von R² und wenigetens ein Rest R¹ die von R³ annimt und
die Einheiten der Formel (II) proteinogene oder nicht proteinogene Aminosäuren sind und zu nicht mehr als 20 Gew.-%, bezogen auf die copolymeren Polyasparaginsäurederivate, enthalten sind.

Alle gegebenen Angaben zur Zusammensetzung der polymeren Produkte beziehen sich wie üblich auf die mittlere Zusammensetzung der Polymerketten.

Die Aminosäurebausteine (II) aus der Gruppe der proteinogenen Aminosäuren leiten sich beispieleweise von Glutaminsäure, Glutamin, Asparagin, Lysin, Alanin, Glycin, Tyrosin, Tryptophan, Serin und Cystein sowie deren Derivaten ab; nicht proteinogene Aminosäuren können beispielsweise β-Alanin, ω-Amino-1-alkansäuren etc. sein.

Die restlichen Einheiten, weiche nicht die Struktur (I) oder (II) haben (nicht mehr als 25 Mol-% aller Einheiten), können unter anderem Iminodisuccinat-Einheiten der Formel (III) 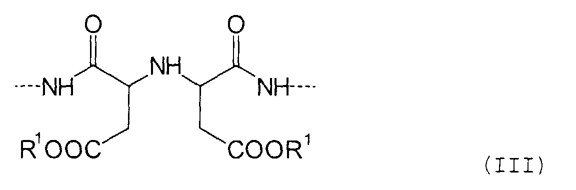 sowie verschiedene Endgruppen sein, am N-Terminus beispielsweise Asparaginsäure-, Maleinsäure-, Fumarsäure- und Äpfelsäureeinheiten sowie deren Ester oder Amide, Maleinimideinheiten oder Diketopiperazine abgeleitet von Asparaginsäure und/oder den Aminosäurebausteinen (II), sowie Ester oder Amide der Aminosäurebausteine (II),am C-Terminus beispielsweise Asparagin- oder Äpfelsäureeinheiten, deren Mono- oder Diester, Amide oder cyclischen Imide.

Erfindungsgemäß bevorzugt sind Verbindungen, bei denen wenigstens eine freie Carboxylatgruppe (R¹ = H, Metall, Ammonium) vorhanden ist, wenigstens ein Rest R³ gleiche oder verschiedene Radikale der Struktur R⁹-X- umfaßt, wobei R⁹ aus der Gruppe der geradkettigen oder verzweigten, gesättigten oder ungesättigten Alkylreste mit 6 bis 30 C-Atomen stammt (z. B. verzweigte oder lineare Octyl-, Decyl-, Dodecyl-, Tetradecyl-, Hexadecyl-, Octadecyl-, Docosylreste, auch ungesättigte und mehrfach ungesättigte Spezies wie z. B. Oleyl) und X eine Polyoxyalkylenkette von 0 bis 100 Alkylenglykoleinbeiten, vorzugsweise abgeleinet von Ethylenoxid, Propylenoxid oder Gemischen daraus ist, und gegebenenfalls Reste R⁴ aus der Gruppe der geradkettigen oder verzweigten, gesättigten oder ungesättigten Alkyl- oder Alkenylreste mit 1 bis 5 C-Atomen enthalten sind (z. B. Methyl, Ethyl, n-Propyl, i-Propyl, n-Butyl, i-Bucyl, s-Butyl, n-Pentyl). Eine weiter bevorzugte Form der Copolymeren enthält Alkyl- oder Alkenylreste R⁹ mit 10 bis 22 C-Atomen ohne Alkylenglykolspacer (Alkylenglykolkettenlänge 0) sowie ggf. geringe Mengen von Alkylresten mit 1 bis 4 C-Atomen.

Derartige Derivate sind beispielsweise durch das in der DE 195 45 678 entsprechend der ER 96 118 806.7 beschriebene Herstellverfahren aus Monoestern monoethylenisch ungesättigter Dicarbonsäuren, beispielsweise Maleinsäuremonoestern und Ammoniak zugänglich.

Die Herstellung der erfindungsgemäßen Polyasparaginsäurederivate kann mit oder ohne Zusatz von organischen Lösungsmitteln erfolgen. Als Lösungsmittel kommen beispielsweise Alkohole, Ketone, Ester, Oligo- und Poly(alkylen)glykole bzw. -glykolether, Dimethylsulfoxid, Dimethylformamid, N,N-Dimethylacetamid oder N-Methylpyrrolidon sowie deren Gemische und andere in Frage. Bevorzugt eingesetzt werden Alkohole mit 2 bis 4 C-Atomen sowie Ketone wie z. B. Methylisobutylketon oder Methylisoamylketon oder Alkylester von Carbonsäuren mit 1 bis 4 C-Atomen, wie beispielsweise Essigsäure-sec-Butylester oder Essigsäurepentylester. Die Reaktion kann gegebenenfalls in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und/oder 0 bis 5 Mol Propylenoxid an C₁₂-C₃₀-Fettalkohole und Wollwachsalkohole; Ethylenoxidanlagerungsprodukte von Glycerinmono- und -diestern und Sorbitanmono- und -diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellungsverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quarternären Ammoniumverbindungen, quarternisierten Proteinhydrolysate, Alkylamidoamine, quarternären Esterverbindungen, quarternären Siliconöle oder quarternären Zucker- und Polysaccharidderivate, anionische Tenside beispielsweise aus der Gruppe der Sulfate, Sulfonate, Carboxylate sowie Mischungen derselben, beispielsweise Alkylbenzosulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisothionate, Ethercarboxylate, Monoalkylphosphate, N-Acylaminosäurederivate wie N-Acylaspartate oder N-Acylglutamate, N-Acylsarcosinate, amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureester, Zuckerester, beispielsweise Fettsäureester der Saccharose, Fructose oder des Methylglucosids, Sorbitolfettsäureester und Sorbitanfettsäureester (gegebenenfalls oxethyliert), Alkyl- oder Alkenylpolyglucoside und deren Ethoxylate, Fettsäure-N-alkylpolyhydroxyalkylamide, Polyglycerinester, Fettsäurealkanolamide, langkettige tertiäre Aminoxide oder Phosphinoxide sowie Dialkylsulfoxide enthalten sein.

Vorzugsweise verbleiben die verträglichkeitsfördernden Agenzien im Produkt. Die Umsetzung zum Copolymeren erfolgt mit wäßrigem oder gasförmigem Ammoniak bei Temperaturen von 20 bis 150 °C sowie nachfolgender Behandlung bei 70 bis 220 °C, vorzugsweise 100 bis 140 °C, unter vermindertem Druck, beispielsweise in Knetapparaturen, Hochviskosreaktoren, Extrudern oder Rührreaktoren, gegebenenfalls unter Einsatz scherkraftreicher Rührer, wie Mig- oder Intermig-Rührer.

Weiterhin können Umsetzungsprodukte von Polyasparaginsäure oder Polysuccinimid mit langkettigen Alkoholen oder die Produkte der Umsetzung von Polyasparaginsäure oder Polysuccinimid mit kurzkettigen Alkoholen, beispielsweise mit 1 bis 5 C-Atomen, mit nachfolgender Umesterung mit langkettigen Alkoholen mit 6 bis 30 C-Atomen eingesetzt werden, ggf. nach abschließender Hydrolyse, beispielsweise mit Alkalimetallhydroxiden. Derartige Derivate sind in DE 195 45 678 und der EP 96 118 806.7 beziehungsweise in DE 195 24 097 A1 beschrieben.

Weitere erfindungsgemäß eingesetzte Polyasparaginsäurederivate sind beispielsweise Copolymere, weiche aus Asparaginsäureeinheiten mit freien Carbonsäure- bzw. Carboxylatseitenketten, aus N-alkylsubstituierten bzw. N,N-dialkylsubstituierten Aspartamideinheiten sowie ggf. aus restlichen Polysuccinimideinheiten bestehen. Solche Produkte sind z. B. durch partielle Ringöffnung von Polysuccinimid (Anhydropolyasparaginsäure) mit. Aminen, beispielsweise geradkettigen oder verzweigten, gesättigten oder ungesättigten C₂-C₂₀ Aminen, vorzugsweise Fettaminen mit 8 bis 18 C-Atomen, zugänglich sowie durch nachfolgende Hydrolyse der restlichen Succinimideinheiten zu Polyasparaginsäureeinheiten bzw. deren Salz. Derartige Verbindungen mit mindestens einem langkettigen N-Alkylrest sind beispielsweise in EP 0 406 623 A oder in DE 22 53 190 A beschrieben oder können leicht durch Hydrolyse der nach DE 40 02 736 A zugänglichen entsprechenden Poly(N-alkylaspartamid-co-succinimide) erhalten werden.

Weiterhin können copolymere Polyasparaginsäurederivate eingesetzt werden, weiche aus Asparaginsäureeinheiten mit freien Carbonsäure- bzw. Carboxylatseitenketten, aus N-hydroxyalkylsubstituierten Aspartamideinheiten sowie deren Acylierungsprodukten mit langkettigen Carbonsäurederivaten und ggf. aus restlichen Polysuccinimideinheiten bestehen. Derartige Verbindungen sind durch Hydrolyse, z. B. alkalische Hydrolyse mit Alkalimetallhydroxiden, der in EP 0 458 079 A beschriebenen copolymeren Polysuccinimidderivate zugänglich.

Weiterhin können copolymere Asparaginsäurederivate eingesetzt werden, welche aus Asparaginsäureeinheiten mit freien Carbonsäure- bzw. Carboxylatseitenketten, aus N-alkylsubstituierten beziehungsweise N,N-dialkylsubstituierten Aspartamideinheiten sowie gegebenenfalls aus Polysuccinimideinheiten bestehen und welche an den das Polyamidrückgrat bildenden Amidgruppen partiell oder vollständig N-Alkylsubstituenten tragen. Derartige Verbindungen sind beispielsweise nach US 5 357 004 A aus Maleinsäure, Ammoniak und Alkylaminen zugänglich.

Weiterhin können copolymere Asparaginsäurederivate eingesetzt werden, weiche aus Asparaginsäureeinheiten mit freien Carbonsäure- bzw. Carboxylatseitenketten, ggf. aus Polysuccinimideinheiten bestehen sowie am Kettenende eingebaute einwertige Alkylalkohole und/oder Alkylamine enthalten. Derartige Verbindungen sind beispielsweise nach EP 0 650 995 A aus Maleinsäure oder Maleinsäureanhydrid, Ammoniak oder Ammoniumderivaten sowie den Alkylaminen und/oder -alkoholen zugänglich.

Die eingesetzten Polymeren können nachbehandelt werden, beispielsweise durch Behandlung mit Aktivkohle oder anderen Adsorbentien, Bleichung mit Oxidationsmitteln wie H₂O, Cl₂, O₃, Natriumchlorit, Natriumhypochlorit etc. oder Reduktionsmitteln wie beispielsweise NaBH₄ oder H₂ in Gegenwart von Katalysatoren.

Die erfindungsgemäßen O/W-Emulsionen zeichnen sich - auch bei Temperaturbelastung - durch eine hohe Lagerstabilität bei konstanter Viskosität aus. Die Eigenschaften der Polyasparaginsäurederivate erlauben dabei oft sogar die Verminderung der Menge an eingesetzten Konsistenzgebern im Vergleich zu üblichen Emulgatoren.

In einer bevorzugten Ausführungsform der Erfindung enthalten die Emulsionen - bezogen auf den Emulgator/Konsistenzgeberanteil - 1 bis 99 Gew.-%, vorzugsweise 5 bis 50 Gew.-% Polyasparaginsäurederivate, 1 bis 99 Gew.-%, vorzugsweise 15 bis 80 Gew.-% eines oder mehrerer Konsistenzgeber, sowie 0 bis 75 Gew.-% weiterer Coemulgatoren. Der nichtwäßrige Anteil der Emulsionen, der sich weitestgehend aus dem Emulgator/Konsistenzgeber sowie dem Ölkörpergehalt zusammensetzt, liegt üblicherweise bei 5 bis 95 und vorzugsweise bei 15 bis 75 Gew.-%. Das bedeutet umgekehrt, daß die Emulsionen 5 bis 95 und vorzugsweise 25 bis 85 Gew.-% Wasser enthalten können, abhängig davon, ob Lotionen mit einer vergleichsweise niedrigen Viskosität oder Cremes und Salben mit einer hohen Viskosität hergestellt werden sollen.

Die erfindungsgemäß in Kombination mit Polyasparaginsäurederivaten eingesetzten Konsistenzgeber können hydrophile Wachse sein, weiche über freie Hydroxyl- oder Carboxylgruppen verfügen und bei Raumtemperatur fest sind. Typische Beispiele sind C₁₂-C₃₀-Fettalkohole, vorzugsweise lineare, gesättigte C₁₆-C₂₂-Fettalkohole, Wollwachsalkohole, gesättigte C₁₆-C₂₂-Fettsäuren, Mono- und Diester von Glycerin, Sorbitan, Ethylenglykol oder Polyalkylenglykolen mit gesättigten C₁₂-C₂₂-Fettsäuren.

Die Emulsionen können als Hautpflegemittel wie beispielsweise in Tagescremes, Nachtcremes, Pflegecremes, Nährcremes, Bodylotions, Salben und dergleichen eingesetzt werden und als weitere Hilfs- und Zusatzstotte Coemulgatoren, Ölkörper Überfettungsmittel, Fette, Wachse, Stabilisatoren, Wirkstoffe, Glycerin, Farb- und Duftstoffe enthalten.

Als weitere Coemulgatoren kommen beispielsweise in Frage: Anlagerungsprodukte von 2 bis 30 Mol Ethylenoxid und/oder 0 bis 5 Mol Propylenoxid an C₁₂-C₃₀-Fettalkohole und Wollwachsalkohole, vorzugsweise lineare, gesättigte C₁₆-C₂₂-Fettalkohole; Ethylenoxidanlagerungsprodukte von Glycerinmono- und -diestern und Sorbitanmono- und -diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose, Polysiloxan-Polyalkyl-Polyether-Copolyrnere und deren Derivate.

Als Coemulgatoren können auch anionische, kationische, nichtionische, amphotere und/oder zwitterionische Tenside, beispielsweise aus der als verträglichkeitsfördernden Agenzien bezeichneten Gruppe ausgewählt sein.

Es können jeweils beliebige Mischungen der o.g. Konsistenzgeber und Coemulgatoren eingesetzt werden.

Als Ölkörper kommen beispielsweise Ester von linearen C₆-C₂₀-Fettsäuren mit linearen C₆-C₂₀-Fettalkoholen, Ester von verzweigten C₆-C₁₃ Carbonsäuren mit linearen C₆-C₂₀-Fettalkoholen, Ester von linearen C₆-C₂₀-Fettsäuren mit Isopropanol oder verzweigten Alkoholen, beispielsweise 2-Ethylhexanol, 2-Hexyldecanol, Ester von linearen und/oder verzweigten C₆-C₂₀-Carbonsäuren mit mehrwertigen Alkoholen (wie beispielsweise Dimerdiol oder Dimertriol) und/oder Guerbetalkoholen, Diester von Dicarbonsäuren wie Adipinsäure, Sebacinsäure, Bernsteinsäure etc. mit linearen oder verzweigten Alkylalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether physiologisch verträgliche lineare oder verzweigte aliphatische bzw. naphthenische Kohlenwasserstoffe und/oder Siliconöle wie cyclische und lineare Polydimethylsiloxane in Betracht. Der Anteil der Ölkörper am nichtwäßrigen Anteil der Emulsionen kann 5 bis 99, vorzugsweise 10 bis 75 Gew.-% betragen.

Als Überfettungsmittel können beispielsweise Lanolin und Lecithinderivate sowie deren Ethoxylate, Polyolfettsäureester, Monoglyceride und Fettsäurealkanolamide verwendet werden. Es können Siliconverbindungen wie Polydimethylsiloxane, Cyclodimethicone sowie amino-, fettsäure-, alkohol-, epoxy-, fluor-, und/oder alkylmodifizierte Siliconverbindungen sowie Wachse wie beispielsweise Bienenwachs, Paraffinwachse oder Mikrowachse enthalten sein. Die Emulsionen können Verdickungsmittel wie Polyacrylsäurederivate oder Polysaccharide wie z. B. Xanthan, Carboxymethycellulose, Hydroxyethylcellulose, kationische Cellulose- oder Stärkederivate, kationische Chitin- oder Chitosanderivate, kationische Siliconpolymere, Copolymere von Diallylammoniumsalzen z. B. mit Acrylamiden, Polyethylenimin enthalten. Weiterhin können anorganische Elektrolyte wie Alkali-, Erdalkalimetall- oder Ammoniumhalogenide wie Natrium, Kalium oder Ammoniumchlorid, -sulfate, -nitrate oder carbonate, oder Metallsalze von Fettsäuren, z. B. Magnesium-, Aluminium- oder Zinkstearat als Stabilisatoren oder Zinksalze der Ricinolsäure als Geruchshemmer enthalten sein. Es können übliche Sonnenschutzwirkstoffe wie Titandioxid, p-Aminobenzoesäure etc., Puffersubstanzen, Antioxidantien, Duftscoffe, Farbstoffe, biogene Wirkstoffe wie Pflanzenextrakte oder Vitaminkomplexe, pharmazeutische Wirkstoffe sowie übliche feuchtigkeitsregulierende Substanzen wie Pyrrolidindion-2-carboxylat und Polyhydroxyverbindungen wie Glycerin, Polyglycerine, Propandiol, Polyethylenglykole, Mono- und Polysaccharide enthalten sein. Weiterhin können die Emulsionen Perlglanzmittel wie Ethylenglykoldistearat, feste anorganische Zusatzstoffe wie Oxide, Talkum, Titandioxid, Kieselgele und Silicate, Tonmineralien etc. sowie die üblichen Konservierungsmittel wie Parabene, Sorbinsäure, Phenoxyethanol und andere enthalten.

Die Emulgierung kann in an sich bekannter Weise, das heißt beispielsweise durch Heiß-, Kalt-, Heiß-Heiß/Kalt- beziehungsweise PIT-Emulgierung erfolgen.

### Beispiele 1 bis 3

### Poly (asparaginsäure-co-alkylaspartat)

Die Herstellung der Polyasparaginsäureester erfolgte in Analogie zur DE 195 45 678 durch Umsetzen der Edukte (Monoethylmaleat, Monoalkylmaleat) in Methylisobutylketon mit 1,0 bis 1,5 Equivalenten an Ammoniakgas und anschließendes Ausdestillieren der Reaktionsmischung i. Vak. bei 110 bis 140 °C für 4 bis 6 h.

| BEISPIEL | ALKYLREST | EDUKT: MOL ALKYLMALEAT | EDUKT: MOL ETHYLMALEAT | PRODUKT: MOL-% ALKYLESTER | PRODUKT: MOL-% ETHYLESTER | MOL-% SÄURE |
|---|---|---|---|---|---|---|
| 1 | Dodecyl | 1,2 | 2,8 | 27 | 5 | 68 |
| 2 | Cetyl | 1,0 | 3,0 | 24 | 5 | 71 |
| 3 | Stearyl | 0,8 | 3,2 | 20 | 7 | 73 |

### Beispiel 4

### Poly (asparaginsäure-co-N-cetylaspartamid) 25 %

Die Herstellung der copolymeren Polyasparaginsäure-Polysuccinimid-Derivate mit Amidgruppen erfolgte nach DE 22 53 190 A.

97 g Polysuccinimid (M_{w} ca. 2000) wurden in 500 ml N,N-Dimethylformamid gelöst, mit 60 g Cetylamin versetzt und 2,5 h bei 60 °C gerührt. Das Lösungsmittel wurde i.Vak. abdestilliert, der Rückstand 1 h mit 130 ml 5N NaOH gerührt, in 1 l Methanol gefällt und abfiltriert. Man erhielt ein Produkt mit 24 Mol-% N-Cetylamidgruppen und 76 % Na-Polyaspartateinheiten in 90 %iger Ausbeute.

### Beispiel 5

### O/W-Emulsionen mit Polyasparaginsäurederivaten

| | |
|---|---|
| Cetylpolyaspartat aus Beispiel 2 (25 % in Wasser, pH 5,5) | 2,0 % |
| Glycerin | 3,0 % |
| Konservierungsmittel | 0,1 % |
| Wasser | 70,4 % |
| Glycerinmonostearat (Tegin® M, Th. Goldschmidt AG) | 4,5 % |
| Tegosoft®CT (Capryl-Caprintriglycerid, Th. Goldschmidt AG) | 20,0 % |

Die wäßrige Phase und die Ölkörper/Glycerinmonossearatmischung wurden bei 70 °C zusamengegeben, intensiv mit einem Rotor-Stator-Homogenisator bearbeitet (SG/220 V, 2 min). Die Emulsion (100 ml) wurde 2 Tage bei 20 °C und 7 d bei 45 °C gelagert. Als Vergleichsemulgator diente Steareth-25 (Teginacid® C, Th. Goldschmidt AG). Die sensorische Bewertung der Proben zeigte bei Beispiel 5 keine Änderung der cremeartigen Konsistenz, beim Vergleich eine Verschlechterung. Die Wasserseparation der W/O-Emulsionen wurde nach 2 d Lagerung bei 20 °C und nach weiteren 7 d Lagerung bei 45 °C bestimmt.

| BEISPIEL | WASSERSEPARATION NACH 2 TAGEN / 20 °C (VOL.-%) | WASSERSEPARATION NACH 7 TAGEN / 45 °C (VOL.-%) |
|---|---|---|
| 5 | < 0,1 % | < 0,1 % |
| Vergleich | 0,5 % | 4 % |

### Beispiel 6

### Sonnenschutzcreme mit Polyasparaginsäurederivaten

| | |
|---|---|
| Poly (asparaginsäure-co-cetylaspartat), nach Beispiel 2, (50 %ig in Wasser, pH 5,5) | 4,0 % |
| Tego® Care 215 (Ceteareth 15, Glycerylstearat, Th. Goldschmidt AG) | 1,0 % |
| Tego® Alkanol 18 (Stearylalkohol, Th. Goldschmidt AG) | 2,0 % |
| Tegosoft® CT (Capryl-Caprintriglycerid, Th. Goldschmidt AG) | 4,5 % |
| Avocadoöl | 5,0 % |
| Methoxyzimtsäureoctylester | 3,0 % |
| Wasser | 3,0 % |
| Glycerin | 3,0 % |
| Titandioxidpulver | 3,0 % |
| Keltrol® F, 5 % in Wasser (Xanthan, Kelco) | 8,0 % |
| Parfumöl, Duftstoffe | |

### Beispiel 7

### Pflege auf O/W-Basis

| | |
|---|---|
| Poly(asparaginsäure-co-stearylaspartat), nach Beispiel 3, (50 %ig in Wasser, pH 5,5) | 4,5 % |
| Tego® Care 450 (Polyglyceryl-3-methylglucosiddistearat, Th. Goldschmidt AG) | 1,0 % |
| Tegin® M (Glycerylstearat, Th. Goldschmidt AG) Tego® Alkanol 18 (Stearylalkohol, | 0,5 % |
| Th. Goldschmidt AG) | 0,3 % |
| Avocadoöl | 12,0 % |
| Tegosoft® CT (Capryl-Caprintriglycerid, Th. Goldschmidt AG) | 9,0 % |
| Glycerin | 3,0 % |
| Wasser | 69,7 % |
| NaOH (10 %) ad pH 5,5 | |

### Beispiel 8

### Feuchtigkeitscreme mit Polyasparaginsäurederivaten

| | |
|---|---|
| Poly(asparaginsäure-co-cetylaspartamid) nach Beispiel 4, (50 %ig in Wasser, pH 5,5) | 3,5 % |
| Poly(asparaginsäure-co-dodecylaspartat), nach Beispiel 1, (50 %ig in Wasser, pH 5,5) | 2,0 % |
| Tego® Care 450 (Polyglyceryl-3-methylglucosiddistearat, Th. Goldschmidt AG) | 1,5 % |
| Tegin® M (Glycerylstearat, Th. Goldschmidt AG) | 1,8 % |
| Tego® Alkanol 18 (Stearylalkohol, Th. Goldschmidt AG) | 1,2 % |
| Tegosoft® DO (Decyloleat, Th. Goldschmidt AG) | 7,0 % |
| Tegosoft® OS (Octylstearat, Th. Goldschmidt AG) | 10,0 % |
| Abil Wax® 9801 (Cetylsiloxan, Th. Goldschmidt AG) | 1,0 % |
| Glycerin | 3,0 % |
| Wasser | 67,0 % |
| Lactil® (Natriumlactatzubereitung, Th. Goldschmidt AG) | 2,0 % |
| Konservierungsstoffe, Duftstoffe | |

### Beispiel 9

### Aftershave Lotion

| | |
|---|---|
| Poly(asparaginsäure-co-cetylspartat), nach Beispiel 2, (50 %ig in Wasser, pH 5,5) | 3,0 % |
| Poly(asparaginsäure-co-dodecylaspartat), nach Beispiel 1, (50 %ig in Wasser, pH 5,5) | 1,0 % |
| Tego® Care PS (Methylglucosidsesquistearat, Th. Goldschmidt AG) | 1,5 % |
| Octyloctanoat | 5,0 % |
| Abil® 350 (Silicon, Th. Goldschmidt AG) | 0,5 % |
| Behenylalkohol | 1,5 % |
| Mineralöl (30 mPas) | 5,0 % |
| Cetiol® B (Dibutyladipat, Henkel) | 5,0 % |
| Bienenwachs | 2,0 % |
| Glycerin | 3,0 % |
| Allantoin | 0,1 % |
| Keltrol® T 2 % in Wasser (Xanthan, Kelco) | 12,5 % |
| Wasser | 59,2 % |
| Panthothenol | 0,5 % |
| Bisabolol | 0,2 % |
| Zitronensäure ad pH 6,5, Duftstoffe, Konservierungsmittel SD Alkohol 39 C | |

## Patentansprüche

1. Kosmetische O/W-Emulsionen, enthaltend ein oder mehrere hydrophob modifizierte Polyasparaginsäurederivate, einen oder mehrere Konsistenzgeber, sowie gegebenenfalls weitere Coemulgatoren und übliche Hilfs- und Zusatzstoffe.

2. Kosmetische O/W-Emulsionen nach Anspruch 1, wobei die Polyasparaginsäurederivate aus einem überwiegend aus Asparaginsäureeinheiten bestehenden Polyaminosäurerückgrat bestehen, weiches partiell durch hydrophobe Alkylgruppen mit 6 bis 30 C-Atomen modifiziert ist.

3. Kosmetische O/W-Emulsionen nach Anspruch 1 oder 2, enthaltend copolymere Polyasparaginsäurederivate, die zu wenigstens 75 Mol-% der vorhandenen Einheiten aus Struktureinheiten der allgemeinen Formeln (I) und (II) 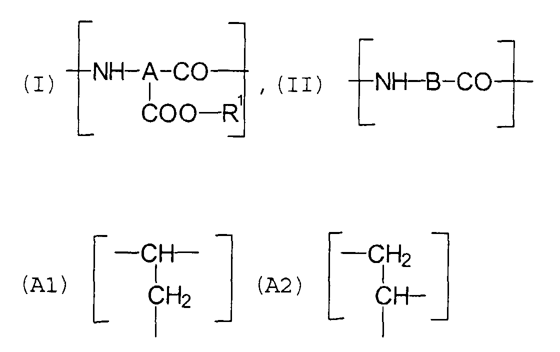 wobei ein Copolymeres aus mindestens drei Einheiten der Formel (I) besteht, worin
R¹ die Bedeutung von R², R³ oder R⁴ hat, wobei
R² ein oder mehrere Reste aus der Gruppe der Alkali-, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Alkylen mit 1 bis 22 C-Atomen oder Hydroxyalkyl mit 1 bis 22 C-Atomen mit 1 bis 6 Hydroxygruppen bedeuten,
R³ gleiche oder verschiedene, geradkettige oder verzweigte, gesättigte oder ungesättigte Alkyl- oder Alkylenreste R⁹ mit 6 bis 30 C-Atomen oder Radikale der Struktur -X-R⁹ sind, wobei X eine Oligo- oder Polyoxyalkylenkette mit 1 bis 100 Oxyalkyleneinheiten ist, und
R⁴ gleiche oder verschiedene, geradkettige oder verzweigte, gesättigte oder ungesättigte Alkyl- oder Alkylenreste mit 1 bis 5 C-Atomen sind,
und wenigstens jeweils ein Rest R¹ die Bedeutung von R² und mindestens ein Rest R² die von R³ annimmt und
die Einheiten der Formel II proteinogene oder nicht proteinogene Aminosäuren sind und zu nicht mehr als 20 Gew.-%, bezogen auf die copolymeren Polyasparaginsäurederivate, enthalten sind.

4. Kosmetische O/W-Emulsionen nach Anspruch 1 oder 2, enthaltend copolymere Polyasparaginsäurederivate, die zu wenigstens 75 Gew.-%, insbesondere zu wenigstens 90 Gew.-%, aus Asparaginsäureeinheiten und/oder deren Metall- oder Ammoniumsalzen sowie aus N-alkylierten und/oder N,N-dialkylierten Aspartamidgruppen bestehen mit wenigstens einem N-Alkylrest mit 6 bis 30 C-Atomen, und gegebenenfalls aus weiteren Polysuccinimideinheiten bestehen.

5. Kosmetische O/W-Emulsionen nach Anspruch 1 oder 2, enthaltend copolymere Polyasparaginsäurederivate, die zu wenigstens 75 Gew.-%, insbesondere zu wenigstens 90 Gew.-%, aus Asparaginsäureeinheiten und/oder deren Metall oder Ammoniumsalzen sowie aus N-alkylierten und/oder N,N-dialkylierten Aspartamidgruppen bestehen, wobei das Polyasparaginsäuerückgrat N-alkylsubstituierte Amidbindungen enthält und mit wenigstens einem N-Alkylrest mit 6 bis 30 C-Atomen und gegebenenfalls aus weiteren Polysuccinimideinheiten bestehen.

6. Kosmetische O/W-Emulsionen nach Anspruch 1 oder 2, enthaltend copolymere Polyasparaginsäurederivate aus Polyasparaginsäureeinheiten, deren Salzen oder Polysuccinimideinheiten, die endständig mit einwertigen Alkoholen oder Aminen mit 6 bis 30 C-Atomen verknüpft sind.

7. Kosmetische O/W-Emulsionen nach Anspruch 1 oder 2, enthaltend oberflächenaktive copolymere Polyasparaginsäurederivate aus Asparaginsäureeinheiten mit freien Carboxyl- und Carboxylatresten, aus N-hydroxyalkylsubstituierten Aspartamideinheiten sowie deren Acylierungsprodukten mit Fettsäurederivaten und gegebenenfalls aus weiteren Polysuccinimideinheiten.

8. Kosmetische O/W-Emulsionen nach einem der Ansprüche 1 bis 3, enthaltend Poly-asparaginsäurederivate, die von den Monoestern α,β-ungesättigter Dicarbonsäuren und Ammoniak, insbesondere von Maleinsäure, Fumarsäure, oder deren Ammoniumsalzen abgeleitet sind.

9. Kosmetische O/W-Emulsionen nach einem der Ansprüche 1 bis 8, dadurch gekennzeichnet, daß sie einen nichtwäßrigen Anteil von 5 bis 95 Gew.-% aufweisen, der nichtwäßrige Anteil 5 bis 99 Gew.-% an Ölkörpern aus der Gruppe der Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe enthält.

10. Kosmetische O/W-Emulsionen nach einem der Ansprüche 1 bis 8, dadurch gekennzeichnet, daß Konsistenzgeber ausgewählt aus der Gruppe der hydrophilen Wachse aus der Gruppe der C₁₂-C₃₀-Fettalkohole, C₁₆-C₂₂-Fettsäuren, Wollwachsalkohole, Glycerinmono- und -diester und Sorbitanmono- und -diester von gesättigten Fettsäuren mit 12 bis 22 C-Atomen oder deren Gemische enthalten sind.

11. Kosmetische O/W-Emulsionen nach einem der Ansprüche 1 bis 8, dadurch gekennzeichnet, daß der Coemulgator ausgewählt ist aus der Gruppe der Anlagerungsprodukte von 2 bis 30 Mol Ethylenoxid und/oder 0 bis 5 Mol Propylenoxid an C₁₂-C₃₀-Fettalkohole und Wollwachsalkohole, der Ethylenoxidanlagerungsprodukte von Glycerinmono- und -diestern und Sorbitanmono- und -diestern von gesättigten und ungesättigten Fettsäuren mit 6 bis 22 C-Atomen, der Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe, der C₁₂-C₁₈-Fettsäuremono- und -diester von Anlagerungsprodukten von 1 bis 30 Mol Ethylenoxid an Glycerin, der Anlagerungsprodukte von Ethylenoxid an Fette und Öle, der Partialester von gesättigten oder ungesättigten C₁₂-C₂₂-Fettsäuren, auch verzweigte oder hydroxysubstituierte, mit Polyolen, Polyalkylenglykolen, Zuckeralkoholen und/oder Polyglucosiden, der Polysiloxan-Polyalkyl-Polyether-Copolymere und deren Derivate, den anionischen Tenside kationische Tenside, nichtionischen Tenside sowie zwitterionischen oder amphoteren Tenside.

12. Verwendung der O/W-Emulsionen nach Anspruch 1 bis 11 als Hautpflegemittel, Tagescreme, Nachtcreme, Pflegecreme, Nährcreme, Bodylotion, pharmazeutische Salbe und Lotion, Aftershave Lotion und Sonnenschutzmittel.
